# EUROPÄISCHE PATENTSCHRIFT

(11) **EP 0 409 048 B1**
(45) Veröffentlichungstag und Bekanntmachung des Hinweises auf die Patenterteilung: **11.01.1995**
(21) Anmeldenummer: 90113143.3
(22) Anmeldetag: 10.07.1990
(51) Int. Cl.: C07D 277/42, C07D 277/40, C07D 417/14, C07D 417/00

(54) **Aminoalkylsubstituierte 2-Aminothiazole und diese enthaltende therapeutische Mittel**
Aminoalkyl substituted 2-aminothiazoles and medicines containing them
2-Aminothiazoles substitués par un aminoalcoyle et médicaments les contenant

(30) Priorität: 18.07.1989 DE 3923675
(43) Veröffentlichungstag der Anmeldung: 23.01.1991
(73) Patentinhaber: BASF Aktiengesellschaft, 67063 Ludwigshafen (DE)
(72) Erfinder: Rendenbach-Mueller, Beatrice, Dr., D-6701 Waldsee (DE); Unger, Liliane, Dr., D-6700 Ludwigshafen (DE); Teschendorf, Hans-Juergen, Dr., D-6724 Dudenhofen (DE)

(56) Entgegenhaltungen:
- EP-A- 0 032 058
- EP-A- 0 345 533
- US-A- 3 210 368
- CHEMICAL ABSTRACTS, Band 95, Nr. 11, 14. September 1981, Seite 663,Zusammenfassung Nr. 97851a, Columbus, Ohio, US;
- & PL-A-106 675 (INSTYTUT PRZEMYSLU FARMACEUTYCZBNEGO) 30-12-1980

## Beschreibung

Die Erfindung betrifft aminoalkylsubstituierte 2-Aminothiazole der allgemeinen Formel I, die wertvolle therapeutische Eigenschaften aufweisen und die sich insbesondere zur Behandlung von Erkrankungen, die mit erhöhtem Blutdruck einhergehen, und von Erkrankungen des zentralen Nervensystems eignen, sowie therapeutische Mittel auf dieser Basis.

In PL 106 675 werden Piperazinylethyl-2-aminothiazole als Neuroleptica beschrieben. Sie wirken Dopamin-antagonistisch.

EP 345 533 beschreibt ähnlich substituierte 2-Aminothiazole u.a. als Dopaminagonisten. Ihre Wirkungen befriedigen jedoch nicht in allen Fällen.

Der Erfindung lag die Aufgabe zugrunde, neue Wirkstoffe mit besseren pharmakologischen Eigenschaften zu entwickeln.

Die Lösung dieser Aufgabe besteht in den aminoalkylsubstituierten 2-Aminothiazolen der Formel I nach Anspruch 1.

In der allgemeinen Formel I 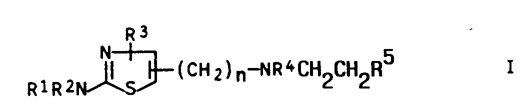
bedeuten
- R¹ und R²,: die gleich oder verschieden sein können, Wasserstoff, C₁-C₅-Alkyl, Phenyl, C₁-C₅-Alkanoyl,
- R³: C₁-C₅-Alkyl, Phenyl, das gegebenenfalls einfach durch Halogen, C₁-C₅-Alkyl oder C₁-C₅-Alkoxy substituiert ist, sowie Thienyl,
- n: eine ganze Zahl von 2 bis 6,
- R⁴: H oder C₁-C₅-Alkyl und
- R⁵: Thienyl oder einen Phenylrest, der gegebenenfalls einfach durch C₁-C₅-Alkyl, C₁-C₅-Alkoxy, Halogen, Hydroxy oder Trifluormethyl substituiert ist,
sowie deren Salze mit physiologisch verträglichen Säuren. Dabei gilt die Maßgabe, daß R³ nur dann C₁-C₅-Alkyl darstellt, wenn R¹ und R² nicht beide gleichzeitig H bedeuten.

Mit Halogen ist Fluor, Chlor, Brom und (weniger bevorzugt) auch Jod gemeint.

Die Verbindungen der allgemeinen Formel I lassen sich beispielsweise herstellen, indem man
a) α-Halogenketone der allgemeinen Formel II, 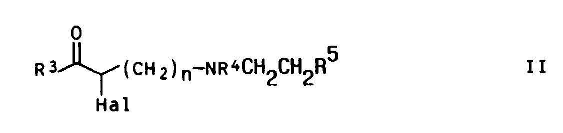 in der R³, R⁴, R⁵ und n wie eingangs definiert sind und Hal ein Chlor-, Brom- oder Iodatom darstellt, oder ein halogenwasserstoffsaures Salz dieser Verbindungen mit einem Thioharnstoff der allgemeinen Formel III, 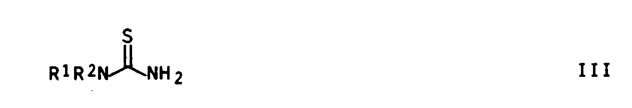 in der R¹ und R² wie eingangs definiert sind, umsetzt,
   oder
b) Verbindungen der allgemeinen Formel IV, 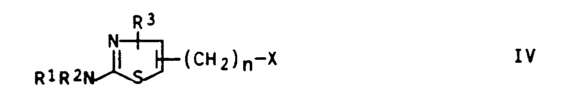 in der R¹, R², R³ und n wie eingangs definiert sind und X für eine nucleofuge Abgangsgruppe wie Chlor, Brom oder R⁸SO₂O steht und R⁸ hierin Niederalkyl oder gegebenenfalls durch C₁₋₃-Alkyl oder Halogen substituiertes Phenyl bedeutet,
   mit einem Amin der allgemeinen Formel V,

   HNR⁴CH₂CH₂R⁵ V

   in der R⁴ und R⁵ die angegebenen Bedeutungen haben, umsetzt,
   oder
c) ein 2-Aminothiazol der allgemeinen Formel VI, 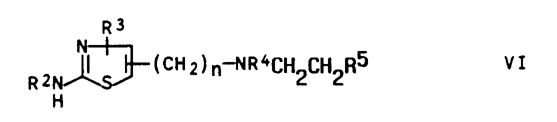 in der R², R³, R⁴, R⁵ und n wie eingangs definiert sind mit einem Alkylierungsreagenz R¹-X oder R¹₂SO₄ oder mit einem Aldehyd RCHO in Gegenwart eines Reduktionsmittels alkyliert oder mit einem Acylierungsmittel 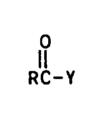 acyliert.

Im Falle des Verfahrens a) erfolgen die Umsetzungen vorzugsweise in einem Lösungsmittel bei Temperaturen zwischen Raumtemperatur und der Siedetemperatur des verwendeten Lösungsmittels, gegebenenfalls in Gegenwart eines säurebindenden Mittels. Als Lösungsmittel können beispielsweise aliphatische Alkohole, Dimethylformamid, Eisessig, Wasser oder ein Lösungsmittelgemisch verwendet werden, als säurebindende Mittel anorganische Basen wie Natrium- oder Kaliumcarbonat oder tertiäre organische Basen wie Triethylamin oder Pyridin. Letztere können, im Überschuß verwendet, gleichzeitig als Lösungsmittel dienen. Das Reaktionsprodukt kann in an sich üblicher Weise gewonnen werden, z. B. durch Filtration, Abdestillieren des Lösungsmittels oder Extraktion aus dem Reaktionsgemisch. Eine Reinigung der erhaltenen Verbindung erfolgt in üblicher Weise, beispielsweise durch Umkristallisieren aus einem Lösungsmittel, Chromatographie oder überführen in eine Säureadditionsverbindung.

Die als Ausgangsstoffe verwendeten α-Halogenketone der allgemeien Formel II erhält man durch Halogenierung der entsprechenden Ketone der allgemeinen Formel VII, 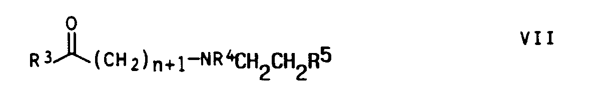
in der R³, R⁴, R⁵ und n die angegebenen Bedeutungen haben. Die Halogenierung erfolgt vorzugsweise mit der äquimolaren Menge Chlor, Brom oder Iod. Die so erhaltenen Verbindungen der Formel II brauchen nicht weiter gereinigt zu werden.

Im Falle des Verfahrens b) erfolgen die Umsetzungen in der Schmelze, gewünschtenfalls auch in Gegenwart eines Lösungsmittels, z. B. Ethylacetat, Tetrahydrofuran, Dimethylformamid, Dimethoxyethan, Toluol oder Xylol bei Temperaturen zwischen Raumtemperatur und dem Siedepunkt des verwendeten Lösungsmittels, vorzugsweise in Gegenwart einer Base wie Natriummethylat, Natriumethylat, Natriumhydrid, Natriumcarbonat, Kaliumcarbonat, eines Amins, z. B. Pyridin. Gegebenenfalls kann auch die Aminkomponente V im Überschuß als Reagenz, Base und Lösungsmittel fungieren. Die als Ausgangsstoffe eingesetzten Verbindungen der Formel IV sind teilweise in der Literatur beschrieben oder können analog hergestellt werden, beispielsweise durch Halogenierung eines ω-Chlorketons der allgemeinen Formel VIII, 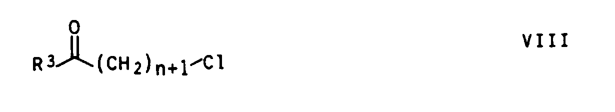
in der R³ und n die angegebenen Bedeutungen haben, und anschließende Umsetzung des halogenierten Ketons mit einem Thioharnstoff-Derivat der Formel III.

Die Umsetzungen nach Verfahren c) verlaufen nach Methoden, wie sie für die Alkylierung und Acylierung von Aminen aus der Literatur bekannt sind. Man kann ohne Gegenwart eines Lösungsmittels die Komponenten miteinander verschmelzen. Es ist aber auch möglich, in Gegenwart eines indifferenten Lösungsmittels, z. B. eines aromatischen Kohlenwasserstoffs wie Toluol oder Xylol, eines Ethers wie Tetrahydrofuran oder Dioxan, eines Ketons wie Aceton oder Butanon, eines Amids wie Dimethylformamid oder N-Methylpyrrolidon oder eines Nitrils wie Acetonitril, umzusetzen. Günstig ist der Zusatz eines säurebindenden Mittels, beispielsweise eines Hydroxids, Carbonats oder eines Amins wie Triethylamin oder Pyridin. Gemäß einer Variante der vorstehenden Methode kann man entsprechend den aus der Literatur für reduktive Aminierungen bekannten Reaktionsbedingungen einen Aldehyd als Alkylierungsmittel unter Zusatz eines geeigneten Reduktionsmittels, vorzugsweise Natriumboranat oder Natriumcyanoborhydrid, einsetzen. Verbindungen der Formel VI können mit einem Acylierungsmittel in die entsprechenden N-Acyl-Derivate übergeführt werden. Als Acylierungsmittel eignen sich insbesondere entsprechende Halogenide (z. B. Acylchloride und -bromide) und Anhydride. Man acyliert vorzugsweise in Lösung bzw. Suspension unter Verwendung inerter Lösungsmittel z. B. von Ethern wie Diethylether, Tetrahydrofuran, Dioxan, Halogenkohlenwasserstoffen wie 1,2-Dichlorethan oder Chlorbenzol, Kohlenwasserstoffen wie Toluol, oder Dimethylformamid, Acetonitril oder Lösungsmittelgemischen, zweckmäßigerweise in Gegenwart einer Base, z. B. eines Alkali- oder Erdalkalihydroxids, eines Alkalicarbonats oder eines Amins. Die Acylierung kann auch mit Estern, vorzugsweise Methylestern der entsprechenden Säuren, durchgeführt werden, zweckmäßigerweise in einem der zuvor genannten Lösungsmittel oder überschüssigem Ester.

Gegebenenfalls werden die erhaltenen erfindungsgemäßen Verbindungen in ihre Säureadditionssalze mit physiologisch verträglichen Säuren überführt. Als übliche physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere können aus Fortschritte der Arzneimittelforschung, Bd. 10, S. 224 f, Birkhäuser Verlag, Basel und Stuttgart, 1966, entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, einem Ether wie Methyl-t-butylether, einem Keton wie Aceton oder Methylethylketon oder einem Ester wie Essigsäureethylester erhalten. Zur besseren Kristallabscheidung können auch Mischungen der genannten Lösungsmittel verwendet werden. Darüber hinaus können pharmazeutisch vertretbare wäßrige Lösungen von Säureadditionsverbindungen der erfindungsgemäßen Verbindungen I durch Auflösen der freien Basen in einer wäßrigen Säurelösung hergestellt werden.

### Rezeptorbindungstest

Striata von Ratten (Sprague Dawley, Charles River) wurden sofort nach Entnahme in 0,32 M Saccharoselösung (0°C) homogenisiert. Das Homogenat wurde filtriert über Gaze, das Filtrat bei 1000 g zentrifugiert (5 min bei 4°C) und der resultierende überstand bei 40000 g zentrifugiert (10 min, 4°C). Der Rückstand (Membranen) wurde in Inkubationspuffer (50 mM Tris-HCl, 1 mM MgCl₂ und 0,1 % Ascorbinsäure, pH 7,4) aufgenommen und 20 min bei 37°C inkubiert. Anschließend wurde durch Resuspension und Rezentrifugation 2x mit Inkubationspuffer gewaschen. Die Membranen wurden portionsweise in flüssigem Stickstoff eingefroren.

Die Testansätze (1 ml) setzten sich zusammen aus Membranen (380 µg Protein), 1 nM ³H-ADTN (NEN, Dreieich Germany, spez. Radioaktivität 1,4 TBq/mmol) und 0,1 µM SCH 23390 (totale Bindung) oder a) mit zusätzlich 50 nM Spiperon (unspezifische Bindung) oder b) mit Prüfsubstanz. Die Ansätze wurden als Triplikate hergestellt.

Nach beendeter Inkubation (40 min bei 25°C) wurden die Ansätze über Glasfaserfilter (Whatman GF/B) filtriert und kurz mit eiskaltem Waschpuffer (Tris-HCl, pH 7,4) gewaschen. Die auf den Filtern zurückgehaltene Radioaktivität wurde mittels Flüssigkeitszintillationsmessung bestimmt. Die unspezifische Bindung betrug ca. 40 - 50 % der totalen Bindung.

Die Auswertung der Kompetitionskurven und die Bestimmung der Dissoziationskonstante erfolgte über iterative nichtlineare Regressionsanalyse in Anlehnung an das Programm "Ligand" (Muson und Rodbard: Anal. Biochem. 107, 220, 1980).

Affinität der Prüfsubstanzen zum Dopamin-D₂-Rezeptor

| Beispiel | Ki (nM) |
|---|---|
| 1 | 20 |
| 2 | 10 |
| 3 | 20 |
| 5 | 4 |

Die erfindungsgemäßen Verbindungen eignen sich zur Bekämpfung von Krankheiten, insbesondere zur Behandlung von Erkrankungen, die mit erhöhtem Blutdruck einhergehen, und Erkrankungen des zentralen Nervensystems (z.B. Parkinsonismus, Schizophrenie). Sie weisen insbesondere wertvolle dopaminerge Wirkungen auf. Die verwendeten Tests zeigen, daß die Substanzen der allgemeinen Formel I dopaminagonistische Aktivität mit Selektivität für präsynaptische Dopaminrezeptoren besitzen. Die Verbindungen der Formel I zeigen hohe Affinität zum D₂-Rezeptor; sie inhibieren die Motilität an Mäusen (gemessen in Lichtschrankenkäfigen) und beeinflussen das Rotationsverhalten an Ratten mit einseitigen 6-Hydroxydopamin-Läsionen der Substantia nigra (Ungerstedt, U. und Abuthnott, G.W., Brain Research 24, 485-493, 1970).

Die erfindungsgemäßen Verbindungen können in üblicher Weise oral oder parenteral (subkutan, intravenös, intramuskulär, intraperitoneal) verabfolgt werden. Die Applikation kann auch mit Dämpfen oder Sprays durch den Nasen-Rachen-Raum erfolgen.

Die Dosierung hängt vom Alter, Zustand und Gewicht des Patienten sowie von der Applikationsart ab. In der Regel beträgt die tägliche Wirkstoffdosis 10 bis 500 mg pro Patient und Tag bei oraler Gabe und 1 bis 500 mg pro Patient und Tag bei parenteraler Gabe.

Die neuen Verbindungen können in den gebräuchlichen galenischen Applikationsformen fest oder flüssig angewendet werden, z. B. als Tabletten, Filmtabletten, Kapseln, Pulver, Granulate, Dragees, Suppositorien, Lösungen oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 1 bis 99 Gew.%.

### Beispiele

### Beispiel 1

### 2-Amino-4-methyl-5-[3-(N-methyl-N-phenethylamino)-propyl]-thiazol-fumarat

25 mmol 2-Amino-5-(3-chlorpropyl)-4-methylthiazol-hydrochlorid, 25 mmol N-Methylphenethylamin, 7 ml Triethylamin, 2,6 g Na₂CO₃ und eine Spatelspitze NaI wurden in 70 ml Ethanol 72 h am Rückfluß gekocht. Die unlöslichen Salze wurden abfiltriert, das Filtrat wurde eingeengt und der Rückstand in i-Propanol gelöst. Der nach Zugabe von 5,2 g Fumarsäure, gelöst, in i-Propanol, ausfallende Feststoff wurde abgesaugt, in Ethanol heiß gelöst, und die unlöslichen Bestandteile wurden abgetrennt. Der nach Abkühlen der Lösung auskristallisierte Feststoff wurde abgesaugt und getrocknet.
Ausbeute: 38 %; Fp. 95-98^{o}C (Zersetzung)
Die folgenden Beispiele wurden analog hergestellt. Die Produkte wurden in Form der freien Base oder eines Salzes mit den üblichen Säuren isoliert.

### Beispiel 2

### 2-Amino-4-methyl-5-[3-(N-phenethyl-N-propylamino)-propyl]-thiazol-fumarat

Es wurden 1,5 Äquivalente N-Propyl-phenethylamin eingesetzt.
Ausbeute: 21 %; Fp. 95-98^{o}C (Zerstäuber; Methyl-t-butylether)

### Beispiel 3

### 2-Amino-4-phenyl-5-[3-(N-propyl-N-phenethylamino)-propyl]-thiazoldihydrochlorid

Es wurden 3 Äquivalente N-Propylmethylamin eingesetzt.
Ausbeute: 41 %; Fp. 95^{o}C (Zersetzung; Essigsäureethylester)

### Beispiel 4

### 2-Acetamino-4-phenyl-5-[3-(N-propyl-N-phenethylamino)-propyl]-thiazoltartrat

Es wurden 2 Äquivalente N-Propylphenethylamin eingesetzt.
Ausbeute: 46 %; Fp. 65^{o}C (Zersetzung; Methyl-t-butylether)

### Beispiel 5

### 2-Amino-4-methyl-5-[4-(N-propyl-N-phenethylamino)-butyl]-thiazol-dihydrochlorid

Es wurden 3 Äquivalente N-Propylphenethylamin eingesetzt.
Ausbeute: 33 %; Fp. 125^{o}C (Zersetzung; Aceton)

### Beispiel 6

### 2-Amino-4-phenyl-5-[4-(N-propyl-N-phenethylamino)-butyl]-thiazol-dihydrochlorid

Es wurden 2,5 Äquivalente N-Propylphenethylamin eingesetzt.
Ausbeute: 29 %; Fp. 80-83^{o}C (Essigsäureethylester)

### Beispiel 7

### 2-Amino-4-methyl-5-[5-(N-propyl-N-phenethylamino)-pentyl]-thiazol-fumarat

Es wurden 3,5 Äquivalente N-Propylphenethylamin eingesetzt.
Ausbeute: 31 %; Fp. 174-175^{o}C (Ethanol)

## Patentansprüche

1. Aminoalkylsubstituierte 2-Aminothiazol-Derivate der allgemeinen Formel I 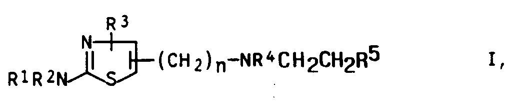 in der
R¹ und R², die gleich oder verschieden sein können, Wasserstoff, C₁-C₅-Alkyl, Phenyl, C₁-C₅-Alkanoyl,
R³ C₁-C₅-Alkyl, Phenyl, das gegebenenfalls einfach durch Halogen, C₁-C₅-Alkyl oder C₁-C₅-Alkoxy substituiert ist, sowie Thienyl,
n eine ganze Zahl von 2 bis 6,
R⁴ H oder C₁-C₅-Alkyl und R⁵ Thienyl oder einen Phenylrest darstellt, der gegebenenfalls einfach durch C₁-C₅-Alkyl, C₁-C₅-Alkoxy, Halogen, Hydroxy oder Trifluormethyl substituiert ist,
mit der Maßgabe, daß R³ nur dann C₁-C₅-Alkyl darstellt, wenn R¹ und R² nicht beide gleichzeitig H bedeuten,
sowie deren Salze mit physiologisch verträglichen Säuren.

2. Verfahren zur Herstellung der aminoalkylsubstituierten 2-Aminothiazol-Derivate gemäß Anspruch 1, dadurch gekennzeichnet, daß man
a) α-Halogenketone der allgemeinen Formel II, 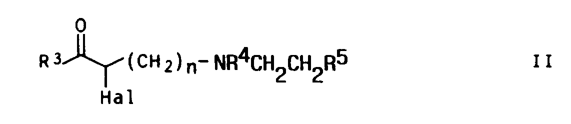 in der R³, R⁴, R⁵ und n wie eingangs definiert sind und Hal ein Chlor-, Brom- oder Iodatom darstellt, oder ein halogenwasserstoffsaures Salz dieser Verbindungen mit einem Thioharnstoff der allgemeinen Formel III, 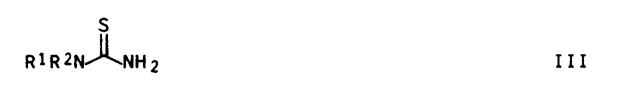 in der R¹ und R² wie eingangs definiert sind, umsetzt,
oder
b) Verbindungen der allgemeinen Formel V, 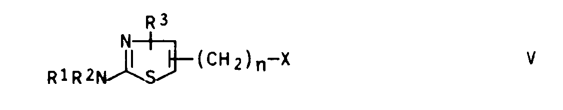 in der R¹, R², R³ und n wie eingangs definiert sind und X für eine nucleofuge Abgangsgruppe wie Chlor, Brom oder R⁸SO₂O steht und R⁸ hierin Niederalkyl oder gegebenenfalls durch C₁₋₃-Alkyl oder Halogen substituiertes Phenyl bedeutet,
mit einem Amin der allgemeinen Formel VI,
HNR⁴CH₂CH₂R⁵ VI
in der R⁴ und R⁵ die angegebenen Bedeutungen haben, umsetzt,
oder
c) ein 2-Aminothiazol der allgemeinen Formel VII, 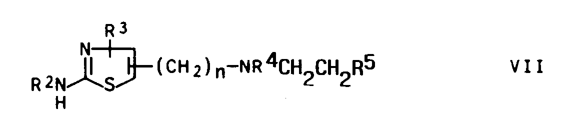 in der R², R³, R⁴, R⁵ und n wie eingangs definiert sind, mit einem Alkylierungsreagenz R¹-X oder R¹₂SO₄ oder mit einem Aldehyd RCHO in Gegenwart eines Reduktionsmittels alkyliert oder mit einem Acylierungsmittel 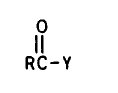 acyliert, wobei R einen C₁-C₄-Alkylrest und Y ein Chloratom oder einen Anhydridrest -O-COR darstellt.

3. Therapeutisches Mittel gegen Erkrankungen, die mit erhöhtem Blutdruck einhergehen, und Erkrankungen des zentralen Nervensystems zur oralen Einnahme, das neben üblichen galenischen Hilfsstoffen 10 bis 500 mg eines 2-Aminothiazolderivates der allgemeinen Formel I nach Anspruch 1 als Wirkstoff enthält.

4. Therapeutisches Mittel gegen Erkrankungen, die mit erhöhtem Blutdruck einhergehen, und Erkrankungen des zentralen Nervensystems zur parenteralen Applikation, das neben üblichen galenischen Hilfsstoffen 1 bis 50 mg eines 2-Aminothiazolderivates der allgemeinen Formel I nach Anspruch 1 als Wirkstoff enthält.

5. Verwendung eines 2-Aminothiazolderivates der allgemeinen Formel I nach Anspruch 1 zur Herstellung eines Arzneimittels.

## Claims

1. An aminoalkyl-substituted 2-aminothiazole derivative of the formula I 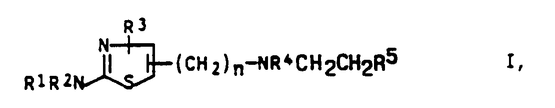 where
R¹ and R², which may be identical or different, are each hydrogen, C₁-C₅-alkyl, phenyl or C₁-C₅-alkanoyl,
R³ is C₁-C₅-alkyl, phenyl which is unsubstituted or monosubstituted by halogen, C₁-C₅-alkyl or C₁-C₅-alkoxy, or thienyl,
n is an integer of from 2 to 6,
R⁴ is H or C₁-C₅-alkyl and R⁵ is phenyl which is unsubstituted or monosubstituted by C₁-C₅-alkyl, C₁-C₅-alkoxy, halogen, hydroxyl or trifluoromethyl or R⁵ is thienyl, with the proviso that R³ is C₁-C₅-alkyl only when R¹ and R² are not both simultaneously H, and its salts with physiologically tolerated acids.

2. A process for the preparation of an aminoalkylsubstituted 2-aminothiazole derivative as claimed in claim 1, wherein
a) an α-haloketone of the formula II 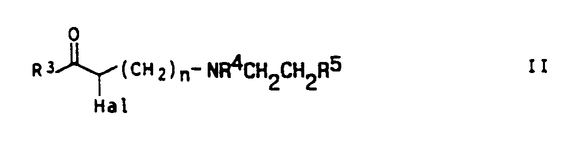 where R³, R⁴, R⁵ and n are as defined at the outset and Hal is chlorine, bromine or iodine, or a salt of this compound with a hydrohalic acid is reacted with a thiourea of the formula III 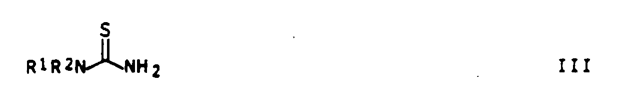 where R¹ and R² are as defined at the outset,
or
b) a compound of the formula IV 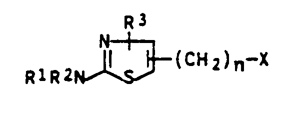 where R¹, R², R³ and n are as defined at the outset and X is a nucleofugic leaving group, such as chlorine, bromine or R⁸SO₂O and R⁸ here is lower alkyl or is phenyl which is unsubstituted or substituted by C₁-C₃-alkyl or halogen, is reacted with an amine of the formula V
HNR⁴CH₂CH₂R⁵ V
where R⁴ and R⁵ have the stated meanings,
or
c) a 2-aminothiazole of the formula VI 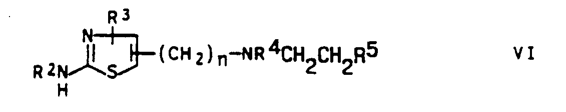 where R², R³, R⁴, R⁵ and n are as defined at the outset, is alkylated with an alkylating reagent R¹-X or R¹₂SO₄ or with an aldehyde RCHO in the presence of a reducing agent or is acylated with an acylating agent 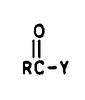 where R is C₁-C₄-alkyl and Y is chlorine or an anhydride radical -O-COR.

3. A therapeutic agent for the treatment of disorders associated with high blood pressure and disorders of the central nervous system, for oral administration, which contains conventional pharmaceutical auxiliaries as well as from 10 to 500 mg of a 2-aminothiazole derivative of the formula I as claimed in claim 1 as an active compound.

4. A therapeutic agent for the treatment of disorders associated with high blood pressure and disorders of the central nervous system, for parenteral administration, which contains conventional pharmaceutical auxiliaries as well as from 1 to 50 mg of a 2-aminothiazole derivative of the formula I as claimed in claim 1 as an active compound.

5. Use of a 2-aminothiazole derivative of the formula I as claimed in claim 1 for the preparation of a drug.

## Revendications

1. Dérivés du 2-aminothiazole aminoalkylsubstitués de la formule générale I : 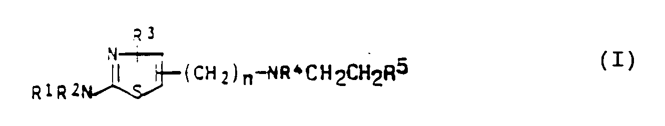 dans laquelle
R¹ et R², qui peuvent être identiques ou différents, représentent chacun un atome d'hydrogène, un radical alkyle en C₁-C₅, phényle, alcanoyle en C₁-C₅,
R³ représente un radical alkyle en C₁-C₅, un radical phényle qui est éventuellement substitué par des halogènes, des radicaux alkyle en C₁-C₅ ou des radicaux alcoxy en C₁-C₅, ainsi qu'un radical thiényle,
n représente un nombre entier dont la valeur varie de 2 à 6,
R⁴ représente un atome d'hydrogène ou un radical alkyle en C₁-C₅, et
R⁵ représente un radical thiényle ou un radical phényle, qui est éventuellement monosubstitué par un groupe alkyle en C₁-C₅, alcoxy en C₁-C₅, un atome d'halogène, un radical hydroxyle, ou un radical trifluorométhyle,
avec la condition que R³ ne représente un radical alkyle en C₁-C₅ qu'alors seulement que R¹ et R² ne représentent pas simultanément des atomes d'hydrogène,
ainsi que leurs sels avec des acides physiologiquement acceptables.

2. Procédé de préparation de dérivés du 2-aminothiazole aminoalkylsubstitués suivant la revendication 1, caractérisé en ce que :
a) on fait réagir des α-halogénocétones de la formule générale II : 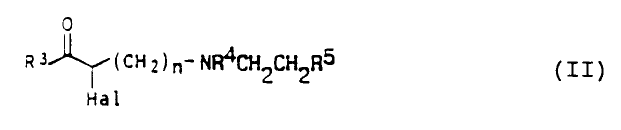 dans laquelle
R³, R⁴, R⁵ et n possèdent les significations qui leur ont été précédemment attribuées et Hal représente un atome de chlore, de brome ou d'iode, ou un sel d'un acide halogènehydrique de ces composés, avec une thiourée de la formule générale III : 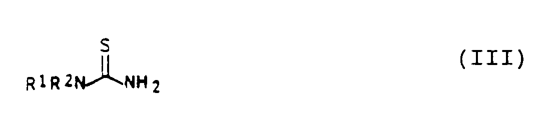 dans laquelle
R¹ et R² sont tels que précédemment définis, ou
b) on fait réagir des composés de la formule générale IV : 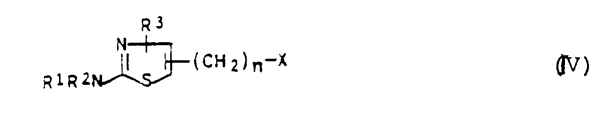 dans laquelle
R¹, R², R³ et n sont tels que précédemment définis et X représente un radical sortant nucléofuge, comme un atome de chlore, de brome ou le groupe R⁸SO₂O, dans lequel R⁸ représente un radical alkyle inférieur ou un radical phényle éventuellement substitué par des radicaux alkyle en C₁-C₃ ou des halogènes,
avec une amine de la formule générale V :
HNR⁴CH₂CH₂R⁵ (V )
dans laquelle
R⁴ et R⁵ possèdent les significations qui leur ont été précédemment attribuées, ou
c) on alkyle un 2-aminothiazole de la formule générale VI : 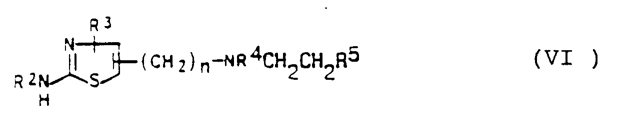 dans laquelle
R², R³, R⁴, R⁵ et n sont tels que précédemment définis, avec un réactif d'alkylation R¹-X ou R¹₂SO₄, ou avec un aldéhyde RCHO, en présence d'un agent de réduction, ou avec un agent d'acylation de la formule : 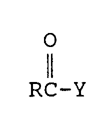 dans laquelle
R représente un radical alkyle en C₁-C₄ et Y représente un atome de chlore ou un reste d'anhydride -O-COR.

3. Agent ou composition thérapeutique contre des maladies associées à une pression sanguine élevée et des maladies du système nerveux central, en vue de l'absorption par la voie orale, qui contient, outre des adjuvants galéniques usuels, de 10 à 500 mg d'un dérivé du 2-aminothiazole de la formule générale I selon la revendication 1, à titre de principe actif.

4. Agent ou composition thérapeutique contre des maladies associées à l'hypertension et des maladies du système nerveux central, pour l'administration parentérale, qui contient, outre des adjuvants galéniques usuels, de 1 à 50 mg d'un dérivé du 2-aminothiazole de la formule générale I selon la revendication 1 à titre de principe actif.

5. Utilisation d'un dérivé du 2-aminothiazole de la formule générale I selon la revendication 1, en vue de la préparation d'un médicament.
